# EUROPEAN PATENT SPECIFICATION

(11) **EP 1 671 132 B1**
(45) Date of publication and mention of the grant of the patent: **27.07.2011**
(21) Application number: 04790172.3
(22) Date of filing: 07.10.2004
(51) Int. Cl.: G01N 33/68

(54) **METHODS FOR SELECTING COMPOUNDS USING ANTIBODIES WITH ACTIVITY AS AGONIST, ANTAGONIST OR ALLOSTERIC MODULATORS**
VERFAHREN ZUR AUSWAHL VON VERBINDUNGEN UNTER VERWENDUNG VON ANTIKÖRPERN MIT AKTIVITÄT ALS AGONIST, ANTAGONIST ODER ALLOSTERISCHE MODULATOREN
PROCEDE DE SELECTION DE COMPOSES UTILISANT DES ANTICORPS AGISSANT COMME AGONISTE, ANTAGONISTE OU MODULATEUR ALLOSTERIQUES

(30) Priority: 10.10.2003 EP 03292525
(43) Date of publication of application: 21.06.2006
(73) Proprietor: Roegel, Jean-Christophe, 68127 Niederentzen (FR); Eftekhari, Pierre, 67200 Strasbourg (FR)
(72) Inventor: Roegel, Jean-Christophe, 68127 Niederentzen (FR); Eftekhari, Pierre, 67200 Strasbourg (FR)
(74) Representative: Gallois, Valérie
(86) International application number: PCT/EP2004/011201
(87) International publication number: WO 2005/040820

(56) References cited:
- EP-A- 1 028 315
- WO-A-01/18051
- WO-A-96/17060
- WO-A-99/40195
- US-A- 6 010 861

## Description

### FIELD OF THE INVENTION

The present invention relates to compositions and methods for screening, identifying, or selecting active compounds against a target protein using peptides derived from said target protein's active site(s) and functional antibodies active on said target protein active site(s).

The invention also relates to the use of these peptides and functional antibodies for screening, identifying or selecting biologically active compounds. We also disclose a kit for implementing the above methods. The target protein is preferably a G-protein-coupled receptor (GPCR), and the invention can be used to identify compounds that are suitable for use in various areas, such as the pharmaceutical, agri-food and cosmetic industries.

### BACKGROUND OF THE INVENTION

Creating a novel drug is an uncertain, time and resource-consuming process consisting of a number of highly selective steps linking timeframes of 8-10 years and costs of 800-1000 M euros. The process involves the selection of a suitable target (i.e. receptor, enzyme, etc.) associated with a given disorder. Drug-like entities interacting with the selected target are identified using complementary approaches, typically a random screening of compound libraries and a structure-based effort, commonly termed rational drug design, and optimized by programs using combinatorial and medical chemistry. The next steps comprise in vitro and in vivo models and gain insight into important parameters such as bioavailability and pharmacokinetics. Altogether, it usually takes several years to identify a clinical candidate, which will then be profiled in humans. Clinical studies consume at least another five years and will determine, after approval by the health authorities, whether the drug candidate will be marketed.

G-protein -coupled receptors (GPCRs) constitute a superfamily of integral membrane proteins encompassing hundreds of receptors for all types of chemical messengers, for example, the key molecules of our light and smell sensory systems, bioactive amines, peptide hormones, neurotransmitters and even proteins. It is the most prominent family of validated drug targets within biomedical research, since approximatively 50 % of approved drugs elicit their therapeutic effects by selectively addressing members of that target family.

Nevertheless, although during the past 10 years or so, associated with the introduction of molecular biology techniques to GPCR research, outstanding progress has been made in understanding the mechanisms of action of these key proteins and their physiological functions, creating a novel drug addressing a GPCR has remained an uncertain, time and resource-consuming process. In fact, from 3D structure point of view, these membrane signal transduction systems represent the most challenging task for structure determination, which is due to the heterogeneous and fine-balanced environmental conditions that are necessary for their structural and functional integrity. GPCRs adapt many different conformations and form homo and hetero-dimers. They have therefore not only an active and an inactive state but many different degrees of activity in between.

Through the years, skilful scientists became able to generate functional antibodies specific not only for a family of GPCRs but also for a given specific isoform. Functional antibodies modify receptor activity and are currently used for characterizing receptors or as pharmaceutical tools for modelling active ligands and/or identifying small organic molecules which possess comparable attributes e.g. by assaying for the ability of potential candidates to compete with functional antibodies on the target protein. Such reporters of binding to a target protein active site (or sites) have been proposed to be selected from a random recombinant antibody library in order to screen for potential drug candidates (EP-A-1028315 and US 6,010,861).

However, in spite of these technical advances, the identification of novel drugs targeting GPCRs stays a long, uncertain and costly task. Indeed, the structure-based screening remains poorly efficient because it does not consider the conformational changes of GPCRs. The screening based on a competitive assay is usually performed in conditions which do not consider all the possible conformations of the target GPCR. All the classical methods (noteworthy mentioned in EP-A-1028315) demand protein isolation and purification or complicated, costly and time-consuming molecular biology techniques. The activity of the target receptor is biased by their artificial conditions, which almost never take into account the native activity of the target receptor; for instance screening based on cells over-expressing a receptor is far from the natural environment of said receptor This is even less probable when it concerns GPCRs, as their activity is dependent on homo and/or hetero-dimerisation. Finally the development of a new expression system based on an isolated protein requires months of full time job. It is uncertain and the validity of the systems which are produced is unpredictable.

As illustration of the prior art,the international WO 99/40195 relates to the use of fragments of antibodies to identity molecules which bind to target protein and the international patent applications WO 96/17060 and WO 01/18051 relate to the use of fragments of target protein as immunogenic fragments in order to obtain specific antibodies.

Therefore, there is a strong need for new (high throughput) methods for selecting active compounds on therapeutic target proteins, more particularly when these targets are GPCRs. All the different active conformations of the therapeutic target proteins should be addressed by these methods.

### SUMMARY OF THE INVENTION

The present invention concerns the method of claims 1 to 25, and the use of claim 26.

The present invention discloses a new universal (high throughput) drug discovery technology using peptides derived from the target protein active site(s) and functional antibodies able to recognise both said target active site(s) and the derived peptide(s), and active on the target protein.

The methods of this invention are designed to be direct and accurate methods for screening drugs acting on biological targets. These methods can be performed on all kinds of extracellular or intracellular biological targets, e.g. receptors (characterized, orphan, resistant to screening, ...) whether ligands are known or unknown. In fact the functional antibody(ies) are active ligand(s) for said biological target. These methods are high throughput methods designed for improving the chance to identify new chemical molecules capable to activate, inhibit, or modulate the function of a specific target (enrichement of ligand diversity). These methods not only accelerate the process of drug discovery and development, but also considerably reduce the resources needed to finalize this process, compared to conventional methods. These methods focus on target's (patho)physiological conformations with functional activity. They address the different conformations of the target protein active's site(s) without requiring any modification of the interacting proteins, like fluorescence or chimeric proteins, nor site-directed mutagenesis.

More specifically, one aspect of the invention discloses a method for *in vitro* screening, identifying, or selecting a molecule active on a target protein using at least one peptide derived from said target protein's active site(s) (including pseudo peptides, reduced, retro and retroinverso peptides) and one functional antibody able to recognise both said target protein's active site(s) and said derived peptide, and active on said target protein. In an other aspect, the invention discloses the use of at least one peptide derived from the target protein's active site(s) (including pseudo peptides, reduced, retro and retroinverso peptides) and one functional antibody able to recognise both said target protein's active site(s) and said derived peptide, and active on said target protein, for *in vitro* screening, identifying, or selecting a molecule active on the target protein. In a more specific embodiment, the functional antibody(ies) is(are) used in combination with a target molecule comprising the epitope(s) of said target protein active site(s) which is(are) recognized by this antibody (or these antibodies) i.e. a peptide (or peptides) derived from the target protein's active site(s) (including pseudo peptides, reduced, retro and retroinverso peptides). We discloses a kit for screening, identifying, or selecting a molecule active on a target protein, comprising at least one functional antibody and a target molecule as defined above not claimed.

In a particular embodiment, the method according to the present invention comprises contacting a candidate molecule with a functional antibody (or functional antibodies) and a target molecule, wherein the target molecule comprises the epitope(s) of the target protein which is(are) recognized by said functional antibody(ies), and determining whether said candidate molecule inhibits or modulates the binding of said functional antibody(ies) to said target molecule. Preferably, inhibition, of the binding of said functional antibody to said target molecule is determined by Inhibition ELISA.

Optionally, the method further comprises the step of testing the capacity of the selected candidate molecule to bind the target molecule in a liquid phase. Optionally, the method further comprises the step of testing the capacity of the selected candidate molecule to interact with said functional antibody. Optionally, the method further comprises the step of testing the capacity of the selected candidate molecule to bind the target protein.

In a preferred embodiment, the method comprises the step of :
(a) contacting a candidate molecule with a functional antibody and a target molecule;
(b) selecting the candidate molecule that inhibits (or modulates) binding of said functional antibody to said target molecule;
(c) testing the capacity of the selected candidate molecule in step b) to bind the target molecule in a liquid phase;
(d) selecting the candidate molecule that inhibits (or modulates) binding of said functional antibody to said target molecule in a liquid phase;
(e) testing the capacity of the selected candidate molecule in step d) to interact with said functional antibody; and,
(f) selecting the candidate molecule(s) which do not bind specifically said functional antibody.

The target protein can be any protein of interest, preferably in a therapeutic approach. Said target protein is typically either an endogenous protein, like an enzyme, a secreted protein or a membrane protein, or an exogenous protein like particles from fungies, bacteries or viruses. More preferably, said target protein is a receptor or a ionic channel. Still more preferably, said target protein is a G-protein-coupled receptor (GPCR).

Preferably, the target molecule comprises, consists essentially of, or consists of a peptide (including pseudo peptides, reduced, retro and retroinverso peptides) derived from the target protein's active site(s). More preferably, the target molecule comprises the sequence of a peptide used as immunogen for preparing the functional antibody(ies). Preferably, the target molecule consists of said peptide.

Preferably, the functional antibody is a polyclonal antibody. Alternatively, the functional antibody is a monoclonal antibody. Optionally, said antibody is an antibody fragment such as Fab, Fab', Fab'2, or an antibody derivative such as a single-chain Fab fragment. humanized antibody, or poly-functional antibody. In a particular embodiment, the functional antibody is prepared by (not claimed):
(a) selecting at least one peptide fragment from (or derived from) the target protein;
(b) raising functional antibodies against said selected peptide;
(c) selecting the raised functional antibodies that are able to bind the peptide and the target protein; and,
(d) selecting the antibodies of c) which are able to modulate the activity of the target protein.

Optionally, the step d) comprises determining whether the raised functional antibodies are agonists, antagonists, or allosteric modulators of the target protein.

Optionally, the functional antibody or the target molecule is immobilized on a solid support. Preferably, the target molecule is immobilized on a solid support, either directly or using a spacer molecule. For example, the solid support is a well, a bead, or a column. The method may comprise the step of :
(a) immobilizing the target molecule on a solid support;
(b) Contacting the immobilized target molecule with a candidate molecule;
(c) adding the functional antibody; and
(d) detecting the binding of said functional antibody on said target molecule.

Preferably, the method is performed on one candidate molecule. Alternatively, the method is performed on a pool of candidate molecules.

Preferably, the method is performed with one couple of a functional antibody / target molecule. Alternatively, the method is performed with several couples of a functional antibody / target molecule.

The methods according to the present invention do not require any modification of the interacting proteins, like fluorescence or chimeric proteins, nor site-directed mutations. They consider all their possible (patho)physiological conformations which improves the possibility to isolate active molecules. Notably immobilising the protein on a solid phase rigidifies its conformations, which in return affect the presentation of the active site(s). Therefore it might hidden the epitope recognised by the functional antibodies and give rise to false negative in the selection of candidate molecules. The latter decreases the number of active conformations and thus the chance of interaction between the target protein's active site and the screened molecules. On the other hand, the constant distribution of the different conformations of the peptide on a solid phase allows a better discrimination between the functional antibodies and candidate molecules. Since the antibody against which the molecule is selected has proven its functional activity and the epitopes presented by the peptide are all covered by functional antibodies, the chance to isolate molecules with pharmacological activity against the target protein increases considerably. Moreover, with this method, the knowledge of at least one GPCR's ligand is not mandatory. Anti-peptide antibodies can be prepared immediately after determining the amino acid sequence of the target protein and particular regions of a protein can be targeted specifically for antibody production; unlimited quantity of pure antigen i.e. synthetic peptide is easily available. Beside being extremely fast, these methods are finally also highly accurate and reproducible. Thus since no particular chemical synthesis or modifications are required the *in vitro* assays can move rapidly over to *in vivo* tests (not claimed). Furthermore, the cost of these methods are far more lower than other methods.

### LEGEND TO THE FIGURES

Figure 1 is a diagram illustrating the Direct ELISA of a functional antibody on the peptide from which the antibody is generated. This Direct ELISA allows the determination of the working dilution for the subsequent assays.
Figure 2 is a diagram illustrating the Direct ELISA of a functional antibody directed against 5-HT₄ receptor on the peptide from which the antibody is generated.
Figure 3 is a diagram illustrating the Indirect ELISA with a functional antibody directed against 5-HT₄ receptor on the peptide from which the antibody is generated in presence of 5-HT, aloprolol, bisoprolol, carbachol, dopamine, forskolin, ICI-118551, isoproterenol, propranolol, or salbutamol.
Figure 4 is a diagram illustrating the Direct ELISA of a functional antibody directed against the M₂ receptor on the peptide from which the antibody is generated.
Figure 5 is a diagram illustrating the Indirect Inhibition ELISA with a functional antibody directed against the M₂ receptor on the peptide from which the antibody is generated in presence or absence of gallamine.

### DETAILED DESCRIPTION OF THE INVENTION

The invention discloses methods and compositions for screening, identifying, or selecting an active compound against a target protein using a peptide or peptides derived from said target protein's active site(s) and functional antibodies able to recognize both said target protein active site(s) and said peptide(s), and active on the target protein i.e. reporters of binding to target protein's active site(s).

In a particular embodiment, the method according to the present invention comprises of allowing contact between a candidate molecule and a functional antibody (or functional antibodies) and a target molecule, wherein the target molecule is a peptide (including pseudo peptides, reduced, retro and retroinverso peptides) which comprises an epitope (or epitopes) of the target protein's active site(s) which is(are) recognized by said functional antibody(ies), and determining whether said candidate molecule inhibits (or modulates) the binding of said functional antibody(ies) to said target molecule.

The methods of the present invention typically comprise a step of testing the capacity of at least one candidate molecule to inhibit (or modulate) the interaction between at least one functional antibody active on the target protein and a target molecule i.e. a peptide (including pseudo peptides, reduced, retro and retroinverso peptides) is derived from the target protein's active site(s) which is(are) recognized by said functional antibody, and selecting the candidate molecules which inhibit (or modulate) said interaction. More particularly, said methods comprise the steps of :
- providing at least one functional antibody active on the target protein;
- providing a target molecule i.e. a peptide (including pseudo peptides, reduced, retro and retroinverso peptides) derived from the target protein's active site(s) which is(are) recognized by that said functional antibody(ies); and,
- testing the capacity of at least one candidate molecule to inhibit (or modulate) the interaction between said functional antibody(ies) and said target molecule.

In a particular embodiment, the peptide derived form the target protein's active site(s) comprises one or several epitopes of the target protein's active site(s).

The candidate molecules which inhibit (or modulate) the interaction between the functional antibody(ies) and its(their) epitope(s) are selected. In a preferred embodiment, the method comprises an additional step of testing the capacity of the selected candidate molecule(s) to interact with the functional antibody. The selected candidate molecule(s) that specifically interact with the antibody, are discarded. In a particular embodiment of the method, the target molecule or the functional antibody is immobilized on a support, typically on the surface of a solid support, such as a bead, well, tube, slide, column, etc., either directly or through spacer groups or molecules. Furthermore, the methods of this invention may further comprise various validation steps, which may be performed subsequently or as alternatives. Such validation steps include (a) assessing the binding of the candidate molecule to the target molecule or protein in a liquid phase, assessing the biological effects of the selected candidate molecule, etc.

In a particular embodiment, the functional antibody directed against the target protein is prepared :
(a) selecting at least one peptide fragment derived from the target protein's active site(s);
(b) raising functional antibodies against said selected peptide(s);
(c) selecting the raised functional antibodies able to bind the peptide(s) and the target protein; and,
(d) selecting the antibodies able to modulate the activity of the target protein.

In a particular embodiment, the step d) comprises determining whether the raised antibodies are agonists, antagonists or allosteric modulators.

### TARGET PROTEIN

The target protein is any protein of interest, preferably in a medical, cosmetic, food or agronomic approach. It may be for instance of human, animal, plant, bacterial or viral origin, most preferably of human or animal origin. Optionally, said target protein can be an extracellular protein, a membrane protein or an intracellular protein. Said target protein can be a circulating protein. Said target protein can be an enzyme. Preferably, said target protein is a membrane protein. More preferably, said target protein is a receptor or a ionic channel. Still more preferably, said target protein is a G-protein-coupled receptor (GPCR). For example, the target protein can be, but is not limited to, a class 1, 2 or 3 GPCR, an enzyme involved in the metabolism of neurotransmitters, both in central and peripheral nervous system, an enzyme involved in the conversion and release of hormones, or an ionic channel (e.g., glutamate, AMPA, NMDA, glycine, GABA, sodium, potassium, chloride, calcium, etc.).

By « molecule active on a target protein » is intended in the present invention any molecule (as defined for the « candidate molecule » below) able to interact with the target protein and modify the activity of the target protein. For example, said molecule is able to increase or decrease the activity of an enzyme, or is an antagonist, an agonist or an allosteric modulator of a target receptor.

By protein's « active site(s) » is intended in the present invention the region(s) of a protein on which a least one molecule (as defined for the « candidate molecule » below) is able to interact and to modify the activity of the target protein. For a GPCR, this site could be in the pharmacophore pocket or outside.

### FUNCTIONAL ANTIBODY

The methods according to the present invention use functional antibodies. As used herein, a functional antibody refers to an antiboby, a fragment thereof or a derivative thereof, which specifically binds a protein and is able to modify the activity of said protein in a biological system of interest (e.g. in vivo) through this binding. For example, the functional antibodies can disturb the binding of a ligand to said protein, modify the conformation of said protein, behave as an agonist, antagonist or an allosteric modulator thereof. Fragments of an antibody include Fab, Fab', Fab'2, etc. Derivatives of an antibody include, within the meaning of this invention, single-chain Fab fragments, humanized antibodies, human antibodies, poly-functional antibodies, etc. Said antibodies can be polyclonal or monoclonal.

A functional antibody beside recognising and binding its target protein, is defined as an antibody which upon its paratope binding (either specifically or in cross reactive interaction) with a target protein's active site alters said target protein activity (said target protein consist either directly or indirectly in an effector system). This alteration is based on the recognition and fixation of antibodies on different states of the target protein. In other word, they are not just reporters of binding to a target active site but preferentially, reporters of binding to the different conformations of the target protein active site(s). This functional antibody stabilises either an active or inactive form of the target protein. A functional antibody is characterised based on its paratope binding to the target molecule and the change induced in the cell's biological activity, in particular in vivo. This could covers the whole area of activity i.e from agonists to antagonists,

Methods for producing polyclonal or monoclonal antibodies, fragments or derivatives thereof, are well known per se to the skilled artisan. More specifically, the following references illustrate the preparation of functional antibodies (Eftekhari et al., 2001, Eur. J. Immunol. 31, 573-579; Sallé et al., 2001, J. Mol. Cell. Cardiol. 33, 405-417 ; Mijares et al., 2000, Mol. Pharmacol. 58, 373-379 ; Fromme et al., 2003, Endocrinology 144, 3262-3269 ; Peter et al., 2003, J Biol Chem.14, in print; Mahler et al., 2001, Eur J Immunol. 31,2210-6 ; Fu et al., 2000, J Hypertension. 18,945-53 ; Mobini et al., 2000, Hybridoma 19,135-42 ; Fu et al.1998, Receptors Channels 6,99-111; Lebesgue et al., 1998, Eur J Pharmacol. 1,348,123-33 ; Elies et al., 1998, Eur J Biochem. 1,251,659-66 ; Ferrari et al., 1995, J Exp Med. 1,182,59-65).

The methods according to the present invention use functional antibodies having high selectivity and activity on the selected target. More preferably, said functional antibodies are produced by a method comprising the following steps:
(a) selecting at least one peptide derived from the target protein's active site(s);
(b) raising antibodies from said selected peptide; and,
(c) selecting the antibodies of b) which are able to modulate the activity of the target protein.

In a more preferred embodiment, the method comprises, prior to or after step c), an additional step of selecting the raised antibodies which are able to bind the peptide and/or the target protein.

Purified or recombinant proteins or fragments thereof can be used to induce functional polyclonal antibodies. Similarly, peptide immobilized on a carrier protein can also be used. However, for the preparation of functional antibodies for use in the present invention, it is preferred to employ, as an immunogen, peptides which are not conjugated to a carrier protein in order to preserve a high degree of liberty. Preferably, the peptide is administered free, but co injected with a carrier protein and/or an appropriate adjuvant.

Peptides, more particularly synthetic peptides, corresponding to region(s) of interest i.e. active site(s) on the target protein, are preferably used as immunogens for preparing the functional antibodies. Preferably, peptides used to generate the antibodies comprise from about 3 to about 100 amino acid residues in length, more preferably from 3 to 35. Synthetic peptides of more than 35 amino acids are not very common, but are not excluded. If peptides longer than 100 amino acids are desirable, different synthetic peptides of between 3-30 amino acid residues in length can be attached to each other, e.g., through non-peptide bonds. The invention also contemplates the use of pseudo peptides, reduced, retro and retroinverso peptides for generating functional antibodies. (Lozano et al., 1998, J Pept Res. 52,457-69; Bolm et al., 2003, Bioorg Med Chem Lett. 13,3207-11; Villemure et al., 2003, Biochemistry, 42, 9659-68; Phan-Chan-Du et al. 2001, Biochemistry, 40,5720-7; Calbo et al., 2000, J Immunother. 23,125-30 ; Calbo et al., 1999, J Immunol. 162,4657-62).

The sequence of the peptide(s) used as immunogen(s) can be selected based on the following criteria.

The candidate peptide should correspond to a domain of the target protein which is accessible to the environment and/or exposed at the surface of the target protein. For a membrane protein, the candidate peptide can be contained within the extracellular or intracellular part of the protein, preferably within the extracellular part, in order to be exposed at the cell surface and directly accessible to antibodies. If the target protein is a receptor, the peptide sequence may also be selected from a region of the receptor which is involved in ligand binding (e.g., binding site, allosteric site) or in modulating the activity of the receptor, preferably upon its interaction with cognate native ligands. For example, the candidate peptide can be derived from an extracellular loop of a GPCR, more particularly from the second extracellular loop. It can also be located in a region of the receptor involved in the interaction with other proteins, particularly with intracellular proteins involved in signal transduction. For an enzyme, the candidate peptide can be selected from a region of the target enzyme which is located at or near the binding site of the substrate, or at or near the catalytic site(s). Most preferably, the peptide is selected from an exposed and functional region of the target protein.

The candidate peptide is preferably specific of the target protein. Accordingly, the peptide preferably lacks (or is essentially devoid of) sequence similarity with other targets from the same family and/or other known proteins involved in identical physiological processes.

Preferably, the global charge of the candidate peptide is such that the charge remains close to the IP value under physiological conditions.

In a specific embodiment, the candidate peptide comprises a B-cell epitope of the target protein. Indeed, peptides containing B-cell epitope(s) usually exhibit high antigenicity, particularly for antibody production. Furthermore, or in the alternative, in order to increase antibody production, the induced immune response can be biased in addition towards one of two known T-helper cell epitopes. In this way, the probability to induce conformational antibodies increases considerably.

One advantage of immunising with a peptide is that unlimited quantity of pure antigen can be used. Anti-peptide antibodies can be prepared immediately after determining the amino acid sequence of a protein and the particular regions of a protein can be targeted specifically for antibody production.

Although less preferred, the entire or part of the target protein may be used as an immunogen to generate functional antibodies.

The polyclonal antibodies can be manufactured according to methods which are known per se, or modifications thereof. For example, a warm-blooded animal is immunized with an immunogen as defined above (e.g., a peptide). Preferably, the immunogen is the peptide as described above, which is co-injected with a carrier protein and an appropriate adjuvant. For example, a carrier protein can be bovine serum albumin, bovine thyroglobulin or hemocyanin. The adjuvant can be for instance complete Freund's adjuvant or incomplete Freund's adjuvant. The administration is usually made once every 2 to 6 weeks and 3 to 10 times in total. The product (e.g., biological fluids such as blood or serum, or tissues or cells) containing or producing the antibodies of the present invention is collected from the immunized animal, followed by separation and purification of the antibodies. Examples of suitable warm-blooded animals are monkeys, rabbits, dogs, guinea pigs, mice, rats, sheep, goats and chickens, with the use of mice and rabbits being preferred.

In the preparation of monoclonal antibodies or their producing cells, warm-blooded animals are immunized with an immunogen as defined above. Immunized animals in which an antibody titer is noted are selected, the spleen or lymph node are collected after two to five days from the final immunization, and antibody-producing cells contained therein are prepared. These cells may be fused with myeloma cells from homozoic or heterozoic animal, to give monoclonal antibody-producing hybridomas. The fusion may be carried out, for example, by the known method described by Kohler and Milstein [(1975), Nature 256, 495-497]. A fusion accelerator may be used, such as polyethylene glycol (PEG), Sendai virus, etc., of which PEG is preferably employed. The monoclonal antibodies can be screened according to known methods or their modifications.

Separation and purification of polyclonal or monoclonal antibodies can be carried out according to known methods, such as for example, salting-out, alcohol precipitation, isoelectric point precipitation, electrophoresis, adsorption and desorption with ion exchangers (e.g., DEAE), ultracentrifugation, gel filtration, or a specific purification method which comprises collecting antibodies with an activated adsorbent such as an antigen, Protein A or Protein G bound to a solid phase.

As indicated above, functional antibodies should be specific for the target protein and bind an active site of said protein and modulate the activity of said protein upon this binding. The antibodies obtained according to the above methods should thus be tested for their specificity for the target protein and for their functionality.

The capacity of an antibody to bind the immunogen or the target protein can be determined according to methods which are known per se in the art, such as enzyme-linked immunosorbent assay (ELISA), radio immuno assay (RIA), electro immuno assay (EIA), nephelometry, turbidimetry, immunoblot, calorimetry, interferometry, immuno-enzymatic assays (IEMA), surface plasmon resonance, etc. More preferably, the interaction between the raised antibodies and either the immunogen or the target protein is detected by mixing the antibody and the immunogen or target protein under conditions allowing interaction to occur, and determining the formation of a complex (i.e., an interaction), typically using a second, labelled antibody specific for the tested antibody(ies). For instance, where the tested antibodies have been produced in mice, the second antibody may be an anti-mouse immunoglobulin or serum. Examples of suitable labels or labeling agents are radioisotopes, enzymes, fluorescent substances, luminescent substances, and the like. Examples of radioisotope are [¹³¹I], [¹³²I], [H], [C], etc. Preferred examples of enzymes are those that are stable and have a high specific activity, which include β-galactosidase, β-glucosidase, alkaline phosphatase, peroxidase, malate dehydrogenase, etc. Examples of fluorescent substances are fluorescamine, fluorescein isothiocyanate, etc. Examples of luminescent substances are luminol, a luminol derivative, luciferin, lucigenin, etc. Furthermore, the biotin-avidin system may also be used.

In a particular embodiment, the capacity of the antibodies to bind the immunogen is tested by ELISA, using both direct and inhibition tests, where the immunogen (or peptide) is immobilised on a support. A target molecule comprising the sequence of the peptide can also be used in ELISA assay. The interaction is inhibited, in a dose dependent manner, with a homologue peptide pre- incubated with antibodies.

For example, the antibodies specific for the native target protein can be tested through western blotting, where the target protein is separated on an acrylamide gel and transferred to a nitro-cellulose membrane. Interaction between the anti-peptide antibodies and the corresponding target protein is revealed. An inhibition test using the target molecule can also be performed.

Futhermore, the antibodies specific for the native target protein can be tested by immunohistochemistry, for determining whether the anti-peptide antibodies are able to recognise the target protein in the tissue containing the native protein. This can be performed on fixed tissue where the target protein is expressed. The antibodies specific for the native target protein can also be tested by immunohistochemistry and fluocytometry on specific cell lines expressing the target protein, either naturally or as a result of transfection.

Moreover, precise parameters of the binding of the antibodies to the target can be determined, for example by fluocytometry, immunohisto-chemistry, immunofluorescence, immunoprecipitation, surface plasmon resonance, tissue blot and 2D western blot. For example, if the target is a receptor, valuable information on the turn over of the receptor due to its interaction with its native ligand or the antibodies can be obtained. These results can be used subsequently in the functional tests.

Once antibodies that specifically bind the target protein or peptide have been identified (or produced), their functionality has to be assessed. To check the functional capacity of the raised antibodies and characterize their nature, in vitro and/or in vivo functional tests can be used. As mentioned above, the functional antibodies should preferably modify the activity of the target protein (e.g., ligand binding, enzymatic activity, signal transduction, etc.). The functional assays depend on the nature of the target protein. Therefore, any assay suitable for testing the activity of the target protein can be used to test the functionality of the raised antibodies. For intracellular targets, functional assays are essentially performed in vitro. For extracellular targets, the functional assays can be performed with cells expressing the target protein, transgenic animals, in vitro, etc. At disposition, beside primary cell cultures, a huge number of human and animal, e.g. rat, mouse, or monkey, cell lines expressing most of already known receptors is available. Preferred assays are performed in order to determine whether the raised antibodies are inhibitors, full, partial or inverse agonists, antagonists or allosteric modulators of a target protein. In a particular embodiment, the effects of the antibodies on the target protein are studied through the measurement of signal transduction within cells, e.g., the intracellular level of calcium (Zambon et al, 2001, Mol Pharmacol. 60, 1375-82; Mijares et al., 2000, Mol Pharmacol, 58, 373-79 ; Buck et al, 1992, J Biol Chem, 267, 23560-7) or of cAMP (Bozon et al, 2002, Receptors Channels, 8, 113-21; Peter et al, 2003, J Biol Chem, 278, 36740-47; Sutherland et al, 1968, Circulation 37,279; Rall et al, 1970, Adv Biochem Psychopharmacol, 3, 113-33), or the pH (Luanna et al, 2003, J Biol Chem, 28, in press; Tominaga et al, 1998, The EMBO Journal, 17, 4712-22).. The raised antibodies can also be tested for apoptose and cell death on cells expressing the target protein. All other suitable assays for assessing the biological function of the antibodies can be used (e.g., competition binding experiment with an endogenous ligand, receptor clustering, receptor internatization, etc.).

Once a functional antibody (i.e., showing specific binding and activity modulation) has been identified or produced, it may be used in the method of this invention, in combination with any corresponding target molecule (i.e., any molecule comprising an epitope of said functional antibody).

### TARGET MOLECULE

The screening of drugs acting on a biological target according to the present invention is carried out by testing the inhibition (or modulation) of the interaction between at least one functional antibody and a target molecule comprisingits epitope. Preferably, said target molecule comprises, consists essentially of or consists of a peptide. More preferably, said target molecule comprises, consists essentially of or consists of an amino acid sequence of a peptide fragment of the target protein used as immunogen for preparing said functional antibody. Still more preferably, said target molecule consists of the peptide used as immunogen. Preferably, the target molecule is a polypeptide comprising from about 5 to about 100 amino acid residues in length, more preferably from 5 to 35 amino acids. More preferably, the target molecule has from about 5 to about 100 amino acid residues in length, more preferably from 5 to 35 amino acids. Synthetic peptides constructed both with L and D amino acids can also be used. The present invention also contemplates the replacement of peptide bonds in the target molecule by a (CH2NH) reduced bond, a (NHCO) retro inverso bond, a (CH2-O) methylene-oxy bond, a (CH2-S) thiomethylene bond, a (CH2CH2) carba bond, a (CO-CH2) cetomethylene bond, a (CHOH-CH2) hydroxyethylene bond), a (N-N) bound, a E-alcene bond or also a -CH-CH- bond. Another possibility is to attach several different synthetic peptides between 5-30 through non-peptide bonds. This improves the possibility to synthetize a peptide target molecule longer than 35, in particular of more than 100 amino acids. The present invention also contemplates pseudo peptides, reduced, retro and retroinverso peptides.

The target molecule may comprise any linear or conformational epitope of the functional antibody. It may, in addition to the epitope, comprise additional residues or moieties, such as amino acids, linkers, chemical groups, functionalised amino acids, etc., which do not alter the peptide sequence and may facilitate its presentation, improve its conformation, stability, attachment, etc. The target molecule can comprise several epitopes of the target protein.

### CANDIDATE MOLECULE

The candidate molecule may be of various origin, nature and composition. It may be any organic or inorganic substance, such as a lipid, peptide, polypeptide, nucleic acid, small molecule, etc., in isolated or in mixture with other substances. The compounds may be all or part of a combinatorial library of products, for instance.

In a particular embodiment, the method is performed with pre-selected candidate molecules. Indeed, a pre-selection step can be performed by selecting the candidate molecule which are able to bind the target molecule. Preferably, the target molecule is immobilized on a solid support or able to be immobilized on a solid support and is contacted with the candidate molecules. Solid support is preferably a bead, more particularly a magnetic bead, or a column.

### METHODS OF SCREENING, SELECTING, IDENTIFYING

The inhibition (or modulation) of the interaction between at least one functional antibody and a target molecule can be tested by various methods known per se in the art.

In a particular embodiment, at least one partner of the reaction, the functional antibodyor the target molecule, is directly or indirectly labelled. In a preferred embodiment, the functional antibody is labelled. The labelling allows the detection of the interaction between the functional antibody and the target molecule. Examples of the labels are radioisotopes, enzymes, fluorescent substances, luminescent substances, and the like. Examples of radioisotopes are [¹³¹I], [¹³²I], [H], [C], etc. Preferred examples of enzymes described above are those that are stable and have a high specific activity, which include β-galactosidase, β-glucosidase, alkaline phosphatase, peroxidase, malate dehydrogenase, etc. Examples of fluorescent substances are fluorescamine, fluorescein isothiocyanate, etc. Examples of luminescent substances are luminol, a luminol derivative, luciferin, lucigenin, etc. Furthermore, the biotin-avidin system may also be used.

In a preferred embodiment of the present invention, at least one partner of the interaction, the functional antibody or the target molecule, is immobilized on a solid support. More preferably, the target molecule is immobilized on a solid support, either directly or using a spacer molecule. The solid support is any suitable support for testing an interaction between the target molecule and the functional antibody. Preferably, the solid support is a well (e.g. 96, 384, 1536 wells plate), a slide, a bead, a tube, a column, a chip, etc ... More preferably, the solid support is a well, a bead, or a column, still more preferably a well.

The interaction between the functional antibody and the target molecule can be tested by a variety of methods which are known per se, such as enzyme-linked immunosorbent assay (ELISA), radio immuno assay (RIA), electro immuno assay (EIA), nephelometry, turbidimetry, immunoblot, calorimetry, interferometry, immuno-enzymatic assays (IEMA), surface plasmon resonance, etc...

In a particular embodiment, the method is performed with several couples of a functional antibody / target molecule. Said antibodies can be directed against peptides derived from the same moiety of the target protein or against peptides derived from different moiety of the target protein. Several antibodies can also be directed against one peptide derived from a moiety of the target protein. In a preferred embodiment of the method of screening, identifying, or selecting an active compound on a target protein according to the present invention, the method is performed with one couple of a functional antibody / target molecule.

In a preferred embodiment, the target molecule is first contacted with the candidate molecule. Then, at least one functional antibody is added. The binding of the antibody to the target molecule is measured. A comparison with the binding of the antibody without the candidate molecule indicates the capacity of the candidate molecule to inhibit the interaction between the functional antibody and the target molecule.

Therefore, in a particular embodiment, the method comprises the step of :
(a) immobilizing the target molecule on a solid support;
(b) contacting the target molecule with a candidate molecule, wherein the target molecule is immobilized on a support;
(c) adding the functional antibody(ies); and
(d) detecting the binding of said functional antibody(ies) on said target molecule.

In a preferred embodiment, the inhibition is tested by ELISA. More preferably, the inhibition is tested by an inhibition ELISA. More particularly, the target molecule is coated on a highly absorbing microtitre plate. Then, non-specific binding is blocked with a blocking buffer. The candidate molecule is contacted with the coated target molecule. At least one functional antibody is added. The binding of the functional antibody(ies) on the coated molecule is then determined. Generally, this binding is measured with a secondary antibody directed against the functional antibody(ies).

Before the inhibition ELISA, a direct ELISA may be performed to determine the dilution at which the value of absorbance due to the interaction between the functional antibody or antibodies and the target molecule is at half max. In this assay, the target molecule is coated on a highly absorbing microtitre plate. Then, non-specific binding is blocked with a blocking buffer. The functional antibody or antibodies is/are contacted with the coated target molecule. The binding of the functional antibody or antibodies on the coated target molecule is then determined. Generally, this binding is measured with a secondary antibody directed against the functional antibody or antibodies.

Preferably, the method further comprises a validation step, to ensure that there is no significant direct interaction between the functional antibody(ies) and the candidate molecule. This assay is preferably a Direct Molecule/Antibody ELISA (DMAE). In this assay, the candidate molecule is coated on a highly absorbing microtitre plate. Then, non-specific binding is blocked with a blocking buffer. The functional antibody or antibodies is/are contacted with the coated candidate molecule. The binding of the functional antibody or antibodies on the coated candidate molecule is then determined. Generally, this binding is measured with a secondary antibody directed against the functional antibody or antibodies. The absence of direct recognition between the functional antibody or antibodies and the test compound is preferred for the selection of the test compound. However, candidate molecules presenting a weak non-specific interaction with the functional antibody(ies) may be selected.

Optionally, the method further comprises an additional or alternative validation step of testing if a candidate molecule, selected in solid phase system, retains the ability to bind the target molecule in liquid phase. Indeed, binding of the target molecule in liquid phase more closely mimics the in vivo conformation of the target protein. This additional step can be performed by a competition experiment, for example in an indirect inhibition ELISA (IIE). More particularly, the target molecule is coated on a highly absorbing microtitre plate. Then, non-specific binding is blocked with a blocking buffer. The candidate molecule is contacted with the coated target molecule. Immediately thereafter, different concentrations of the target molecule are added. Then, the functional antibody or antibodies are added. The binding of the functional antibody or antibodies on the coated candidate molecule is then determined. Generally, this binding is measured with a secondary antibody directed against the functional antibody or antibodies.

In a particular embodiment of the method of screening, identifying, or selecting an active compound on a target protein according to the present invention, the method comprises the step of :
(a) contacting a candidate molecule with a functional antibody or antibodies and a target molecule;
(b) selecting the candidate molecule that inhibits (or modulate) binding of said functional antibody(ies) to said target molecule;
(c) testing the capacity of the selected candidate molecule in step b) to bind the target molecule in a liquid phase;
(d) selecting the candidate molecule that inhibits (or modulate) binding of said functional antibody(ies) to said target molecule in a liquid phase;
(e) testing the capacity of the selected candidate molecule in step d) to interact with said functional antibody(ies); and,
(f) selecting the candidate molecule(s) which do not specifically bind said functional antibody(ies).

Number of variations can be developed by the skilled artisan based on available and well known methods.

In a particular embodiment of the method of screening, identifying, or selecting an active compound on a target protein according to the present invention, the method is performed with a pool of candidate molecules. Once the pool is selected as having the capacity to inhibit the interaction between a functional antibody and its epitope, the method comprises the identification and/or the isolation of the candidate molecule(s), within the pool, having this capacity. In a preferred embodiment, the candidate molecule(s) having this capacity is/are isolated by contacting the selected pool of candidate molecules with the target molecule immobilized on a solid support or able to be immobilized on a solid support. Solid support is preferably a bead, more particularly a magnetic bead, or a column.

In further validation steps, the selected molecules may be characterised in vitro and/or ex vivo for their capacity to bind the target protein, for their specificity for the target protein, and/or their functionality, similarly to the functional antibody or antibodies. In addition, the molecules can be characterized on in vivo models, to evaluate their pharmacodynamic profile and gain insight into important parameters such as safety, tolerability and pharmacokinetics in vivo, i.e., confirm their therapeutic potential (not claimed).

The methods of the present invention can be also used to characterize synthetic potential leads derived from rationalising structure-activity data and lead optimization. For example, the method of screening according to the present invention can be used for optimizing a molecule. Indeed, from one or several selected molecules, several other molecules can be designed and prepared. These molecules can be tested by the method according to the present invention. In this regard, in a particular embodiment, the invention relates to a method of optimizing biologically active compounds, comprising (i) testing a candidate molecule in a method as described above, (ii) preparing derivatives of said molecule and (iii) testing said derivatives in a method as described above, to identify compounds having an improved or optimized activity.

In fact functional antibodies gain also insight into important structure-activity parameters that facilitate lead optimization.

A particular embodiment of the present invention discloses the use of at least one peptide derived from target protein's active site(s) for *in vitro* screening, identifying, or selecting reporters of binding to said target active site(s) from a random antibody library (EP-A-1028315). A pre-selection step can be performed by selecting the candidate antibodies which are able to bind the target molecule.

Further aspects and advantages of the present invention will be disclosed in the following experimental section, which should be regarded as illustrative and not limiting the scope of the present invention.

### EXAMPLES

### Example 1: General description of the HTS screening method using ELISA according to the present invention.

The potential inhibition of the interaction between functional anti-peptide antibodies and the parent peptide derived from a target protein is tested on different forms of ELISA. These assays comprise of Direct ELISA (DE), Direct Molecule/Antibody ELISA (DMAE), Inhibition ELISA (IE) and Indirect Inhibition ELISA (IIE).

### Direct ELISA (DE)

This method is used systematically in order to known at which dilution the value of absorbance at 405 nm, due to the interaction between the antibody and peptide is at half max (AD₅₀ = Active dilution at half max). The desired AC₅₀ value should be between 0,7 -0,8).
- Coating
   Peptide is coated in its appropriate buffer at already calibrated concentration (see appendix 2) on a microtitre high absorption plate for an appropriate time and at an optimal temperature.

- Blocking of non-specific binding
- Wash

The plate is washed with washing buffer (appendix 1).
- First antibody
   Different dilutions of rabbit polyclonal anti-target protein antibodies, in blocking buffer, are subjected to the plate and incubated for an appropriate time and at an optimal temperature.
- Wash
   The plate is washed with washing buffer.
- Second antibody
   Peroxidase conjugated goat anti-rabbit antibodies diluted at appropriate dilution (appendix 2) is allowed to react with the bound polyclonal anti-target protein antibodies for an appropriate time and at an optimal temperature.
- Wash
   The plate is washed three times with washing buffer followed by 3 washes with PBS (appendix 1).
- Revealing
   Bound antibodies are revealed using revealing buffer for a fix period of time (appendix 1 and 2).

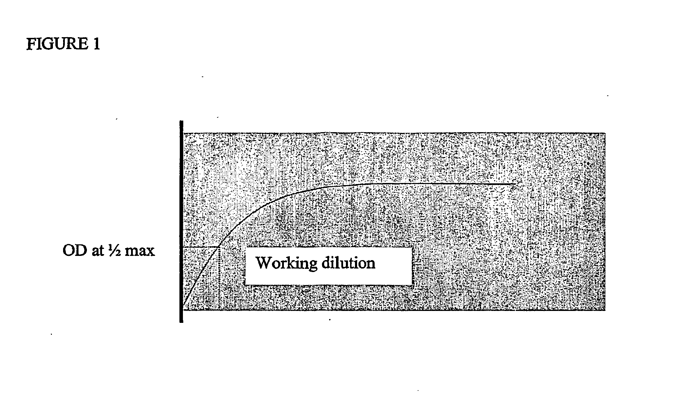

These results are plotted and dilution at AD₅₀ is determined for IE assay (Figure 1)

### Inhibition ELISA (IE)

- Coating
   Corresponding peptide is dissolved extemporaneously in appropriate buffer. Peptide at calibrated concentration is coated on a highly absorbing microtitre plate.

- Blocking of non-specific binding
- Wash

The plate is washed with washing buffer (appendix 1).
- Molecules to be screened on the peptide
   Each molecule is dissolved and distributed on the plate. Immediately first anti-target protein antibody at AD₅₀ dilution is added to all the wells and incubated for an appropriate time and at an optimal temperature.
- Wash
   The plate is washed with washing buffer (appendix 1).
- Secondary antibodies
   Peroxidase conjugated goat anti-rabbit antibodies diluted at an appropriate dilution (appendix 2) is allowed to react with the bound polyclonal anti-GPCR antibodies for an appropriate time and at an optimal temperature.
- Wash
   The plate is washed three times with washing buffer followed by 3 washes with PBS (appendix 1).
- Revealing
   Bound antibodies are revealed using revealing buffer for a fixed period of time (appendix 1 and 2).

Each molecule which is able to decrease the absorbance at 405 nm at around 20% from its control value is selected for the DMAE.

### Direct Molecule/Antibody ELISA (DMAE)

This assay is used to be sure that there is no direct interaction between the antibody and selected molecule.
- Coating
   Corresponding molecule is dissolved extemporaneously. in coating buffer in a known concentration and coated on a highly absorbing microtitre plate for an appropriate time and at an optimal temperature.

- Blocking of on-specific binding
- Wash

The plate is washed with washing buffer (appendix 1).
- First antibody
   Different dilutions of rabbit polyclonal anti-target protein antibodies, in blocking buffer are subjected to the plate and incubated for an appropriate time and at an optimal temperature.
- Wash
   The plate is washed with washing buffer (appendix 1).
- Secondary antibodies
   Peroxidase conjugated goat anti-rabbit antibodies diluted at appropriate dilution (appendix 2) is allowed to react with the bound polyclonal anti-target protein antibodies for 1h at 37°C.

- Wash
- The plate is washed three times with washing buffer followed by 3 washes with PBS (appendix 1).Revealing
   Bound antibodies are revealed using revealing buffer for a fixed period of time (appendix 1 and 2).

The absence of direct recognition between antibody and selected molecule will bring the molecule forward to the next step.

### Indirect Inhibition ELISA (IIE)

The selected molecule should beside binding the parent peptide in solid phase also be able to bind it in liquid phase. The latter would mime the in vivo conformation of the parent peptide.
- Coating
   Corresponding peptide is dissolved extemporaneously in appropriate buffer. Peptide at calibrated concentration is coated on a highly absorbing microtitre plate.
- Blocking of on-specific binding
- Wash

The plate is washed with washing buffer (appendix 1).
- Selected molecules to be tested on IIE
   Each molecule dissolved at known concentration is distributed on the plate. Immediately different cascade concentration of the parent peptide is added to the well. This is thereafter followed by the first antibody at AD₅₀ dilution and incubated for an appropriate time and at an optimal temperature.
- Wash
   The plate is washed with washing buffer (appendix 1).
- Secondary antibodies
   Peroxidase conjugated goat anti-rabbit antibodies diluted at appropriate dilution (appendix 2) is allowed to react with the bound polyclonal anti-GPCR antibodies for an appropriate time and at an optimal temperature.
- Wash

The plate is washed three times with washing buffer followed by 3 washes with PBS (appendix 1).
- Revealing
   Bound antibodies are revealed using revealing buffer for a fixed period of time (appendix 1 and 2).

### Appendix 1

### Working/Washing Buffers

PBS
NaCl 150mM
Na₂HPO₄
KCl
KH₂PO₄
PH = 7.4

### Appendix 2

### Calibration

In order to determine the optimal immunological conditions for ELISA tests, different concentrations of peptides (from 10 ng/ml to 1µg/ml) are coated for 30 min and 1 h on a microtitre plate. After blocking the non specific reactions, different dilutions of corresponding anti-peptide antibodies (1/50 to 1/1000) are incubated with the peptide. Peroxidase conjugated secondary antibodies at 3 different concentrations (1/5000, 1/10000,1/20000) are allowed to react before revealing the plate. Immunochemical interaction which gives an absorbance of 1,000 is the optimum assay condition.

### Examples 2 : HTS screening method usine ELISA according to the present invention for serotoninergic receptor 5-HT_{4.}

The second extracellular loop (SEL) of 5-HT₄ receptor is involved in activating its messenger system, a Gₛ protein, by binding serotonin. Polyclonal antibodies raised in rabbit against the SEL of the 5-HT₄ receptor bind specifically the receptor and inhibit its activation by hindering serotonin to bind to the same binding site. (Eftekhari et al. (2001) Eur. J. Immunol., 31, 573-579)

### Direct ELISA (DE)

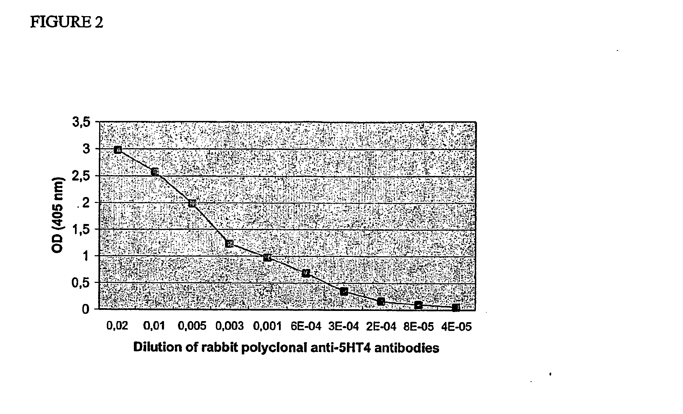

The same protocol as mentioned above in "Example 1" was performed:
Five µg/ml of corresponding peptide (C15Q) was coated on a microtitre plate (Falcon, 96 wells). After blocking nonspecific binding sites with blocking buffer, polyclonal anti-C15Q antibodies at decreasing serial dilutions were allowed to react with the peptide. The complex peptide antibody was revealed with an appropriate second antibody. The results are showed in Figure 2.

### Inhibition ELISA (IE)

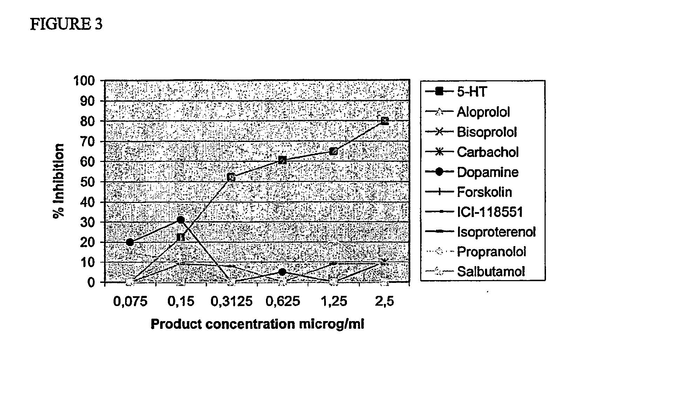

The same protocol as mentioned above in in "Example 1" was performed:
The results are showed in Figure 3.
The interaction of an anti-peptide antibody raised against the 5-HT4 receptor with the corresponding synthetic peptide is specifically inhibited by its native ligand 5-HT. This argues at one hand that the second extracellular loop of the 5-HT4 receptor is one of the binding site for 5-HT and at the other hand that 5-HT can compete with a specific antibody to react with the synthetic peptide. Finally no other irrelevant ligands, specific either for β-adrenergic, dopaminergic and muscarinic receptors are able to mimic this reaction, which shows the specificity of the interaction.

### Example 3 : HTS screening method using ELISA according to the present invention for muscarinic receptor M₂₋

A monoclonal antibody against the SEL of the M₂ receptor with functional activity has been isolated. The isolated monoclonal antibody recognizes specifically the SEL of the M₂ receptor. It activates M₂ receptor at the same site as gallamine. (Elies et al., 1998, Eur J Biochem. 1,251,659-66)

### Direct ELISA (DE)

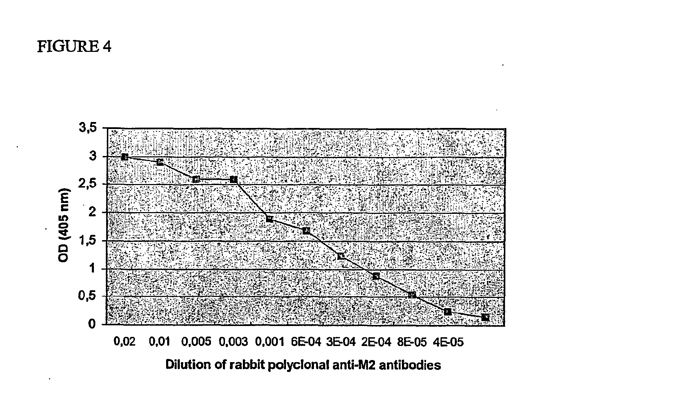

The same protocol as mentioned above in "Example 1" was performed. The results are showed in Figure 4.

### Indirect Inhibition ELISA (IIE)

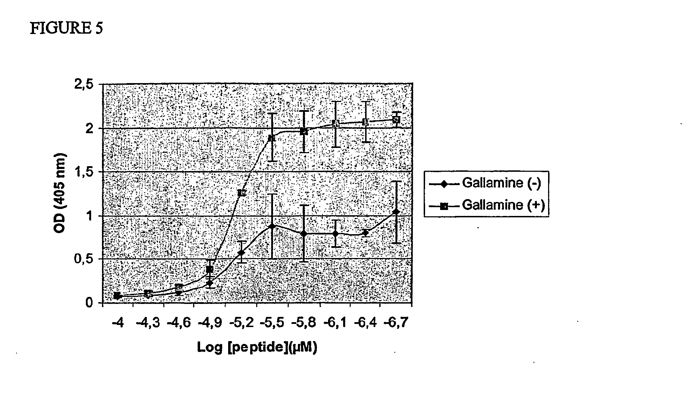

The same protocol as mentioned above in in "Example 1" was performed:
The results are showed in Figure 5.
It is of extreme importance to show that a specific known or candidate ligand for a given receptor is able to compete with the homologue peptide for binding the related anti-peptide antibodies. The difference here with IE is that all three competitors are in liquid phase, i.e. anti-peptide antibodies, homologue peptide and synthetic ligand or molecule to be tested. Figure 5 demonstrates clearly that gallamine which binds specifically the second extracellular loop of the muscarinic M₂ receptor is able to compete with the homologue peptide and hinder the recognition between the homologue peptide and the anti-peptide in liquid phase.

## Claims

1. A method for screening, selecting or identifying a molecule active on a target protein, wherein the method comprises contacting a candidate molecule, a functional antibody or functional antibodies that is or are able to modify the activity of said target protein, and a target molecule being a fragment of the target protein and having from 5 to 100 amino acid residues in length, wherein the target molecule comprises one or several epitopes of said target protein active site(s) which is/are recognized by said functional antibody or antibodies, and determining whether said candidate molecule inhibits binding of said functional antibody or antibodies to said target molecule.

2. Method according to claim 1, wherein said target molecule has from 5 to 35 amino acid residues in length.

3. Method according to claim 1 or 2, wherein said target molecule comprises or consists in an amino acid sequence of a peptide fragment of the target protein used as immunogen for preparing said functional antibody.

4. Method according to any one of preceding claims, wherein said functional antibody or said target molecule is immobilized on a solid support.

5. Method according to claim 4, wherein said target molecule is immobilized on a solid support, either directly or using a spacer molecule.

6. Method according to claim 4 or 5, wherein the solid support is a well, a bead, or a column.

7. Method according to claim 5 or 6, wherein the method comprises the step of :
(a) immobilizing the target molecule on a solid support;
(b) contacting the immobilized target molecule with a candidate molecule;
(c) adding the functional antibody; and
(d) detecting the binding of said functional antibody on said target molecule.

8. Method according to any one of claims 1-7, wherein said target protein is an enzyme.

9. Method according to any one of claims 1-7, wherein said target protein is a membrane protein.

10. Method according claim 9, wherein said membrane protein is a receptor.

11. Method according to claim 10, wherein said receptor is a G-protein-coupled receptor (GPCR).

12. Method according to any one of claims 1-11, wherein said antibody is a monoclonal antibody.

13. Method according to any one of claims 1-11, wherein said antibody is a polyclonal antibody.

14. Method according to any one of claims 1-11, wherein said antibody is an antibody fragment such as Fab, Fab', Fab'2, CDR regions or an antibody derivative such as a single-chain antibody, humanized antibody, or poly-functional antibody.

15. Method according to any one of preceding claims, wherein said functional antibody is prepared by:
a. selecting at least one peptide fragment from the target protein;
b. raising antibodies from said selected peptide;
c. selecting the raised antibodies that are able to bind the peptide and/or the target protein; and,
d. selecting the antibodies of c) which are able to modulate the activity of the target protein.

16. Method according to claim 15, wherein the step d) comprises determining whether the raised antibodies are agonists, antagonists, or allosteric modulators of the target protein.

17. Method according to any one of claims 1-16, wherein the method is performed on one candidate molecule.

18. Method according to any one of claims 1-16, wherein the method is performed on a pool of candidate molecules.

19. Method according to any one of claims 1-18, wherein the method is performed with one couple of a functional antibody / target molecule.

20. Method according to any one of claims 1-18, wherein the method is performed with several couples of a functional antibody / target molecule.

21. Method according to any one of claims 1-20, wherein the method further comprises the step of testing the capacity of the selected candidate molecule to bind the target molecule in a liquid phase.

22. Method according to any one of claims 1-21, wherein the method further comprises the step of testing the capacity of the selected candidate molecule to interact with said functional antibody.

23. Method according to any one of claims 1-22, wherein inhibition of the binding of said functional antibody to said target molecule is determined by Inhibition ELISA.

24. Method according to any one of claims 1-23, wherein the method further comprises the step of testing the capacity of the selected candidate molecule to bind the target protein.

25. Method according to any one of claims 1-24, wherein the method comprises the step of:
(a) contacting a candidate molecule with a functional antibody and a target molecule;
(b) selecting the candidate molecule that inhibits binding of said functional antibody to said target molecule;
(c) testing the capacity of the selected candidate molecule in step b) to bind the target molecule in a liquid phase;
(d) selecting the candidate molecule that inhibits binding of said functional antibody to said target molecule in a liquid phase;
(e) testing the capacity of the selected candidate molecule in step d) to interact with said functional antibody; and,
(f) selecting the candidate molecule(s) which do not bind specifically said functional antibody.

26. Use of at least one functional antibody that is able to modify the activity of said target protein, directed against a target protein and at least one target molecule being a fragment of the target protein, having from 5 to 100 amino acid residues in length and comprising one epitope of said target protein which is recognized by said functional antibody for screening, selecting or identifying a molecule active on said target protein.

## Patentansprüche

1. Ein Verfahren zur Suche, Auswahl oder Identifizierung eines Moleküls, das auf ein Zielprotein wirkt, wobei das Verfahren das in Kontakt bringen eines Kandidatenmoleküls, eines funktionalen Antikörpers oder funktionaler Antikörper und eines Zielmoleküls und das Bestimmen umfasst, ob das Kandidatenmolekül die Bindung des funktionalen Antikörpers oder der funktionalen Antikörper an das Zielmolekül inhibiert, wobei der/die funtionale/n Antikörper in der Lage ist/sind, die Aktivität des Zielproteins zu modifizieren und das Zielmoleküls ein Fragment des Zielproteins ist und eine Länge von 5 bis 100 Aminosäureresten aufweist, wobei das Zielmolekül ein oder mehrere Epitope der Zielprotein aktiven Stelle(n), die durch den funktionalen Antikörper oder die funktionalen Antikörper erkannt wird/werden, umfasst.

2. Verfahren nach Anspruch 1, wobei das Zielmolekül eine Länge von 5 bis 35 Aminosäureresten aufweist.

3. Verfahren nach Anspruch 1 oder 2, wobei das Zielmolekül eine Aminosäuresequenz eines Peptidfragments des Zielproteins, das als Immunogen für die Herstellung des funktionalen Antikörpers verwendet wird, umfasst oder aus dieser besteht.

4. Verfahren nach einem der vorhergehenden Ansprüche, wobei der funktionale Antikörper oder das Zielmolekül an einen festen Träger immobilisiert sind.

5. Verfahren nach Anspruch 4, wobei das Zielmolekül entweder direkt oder unter Verwendung eines Spacermoleküls an einen festen Träger immobilisiert ist.

6. Verfahren nach Anspruch 4 oder 5, wobei der feste Träger eine Vertiefung, eine Kugel oder eine Säule ist.

7. Verfahren nach Anspruch 5 oder 6, wobei das Verfahren die folgenden Schritte umfasst:
(a) Immobilisieren des Zielmoleküls an einen festen Träger;
(b) In Kontakt bringen des immobilisierten Zielmoleküls mit einem Kandidatenmolekül;
(c) Hinzufügen des funktionalen Antikörpers; und
(d) Detektieren der Bindung des funktionalen Antikörpers an das Zielmolekül.

8. Verfahren nach einem der Ansprüche 1 bis 7, wobei das Zielprotein ein Enzym ist.

9. Verfahren nach einem der Ansprüche 1 bis 7, wobei das Zielprotein ein Membranprotein ist.

10. Verfahren nach Anspruch 9, wobei das Membranprotein ein Rezeptor ist.

11. Verfahren nach Anspruch 10, wobei der Rezeptor ein G-Protein gekoppelter Rezeptor (GPCR) ist.

12. Verfahren nach einem der Ansprüche 1 bis 11, wobei der Antikörper ein monoklonaler Antikörper ist.

13. Verfahren nach einem der Ansprüche 1 bis 11, wobei der Antikörper ein polyklonaler Antikörper ist.

14. Verfahren nach einem der Ansprüche 1 bis 11, wobei der Antikörper ein Antikörperfragment wie Fab, Fab', Fab'2, CDR-Regionen oder ein Antikörperderivat wie ein einzelkettiger Antikörper, humanisierter Antikörper oder polyfunktionaler Antikörper ist.

15. Verfahren nach einem der vorhergehenden Ansprüche, wobei der funktionale Antikörper hergestellt wird durch:
a. Auswählen von mindestens einem Peptidfragment des Zielproteins;
b. Anziehen von Antikörpern gegen das ausgewählte Peptid;
c. Auswählen der angezogenen Antikörper, die in der Lage sind, das Peptid und/oder das Zielprotein zu binden, und,
d. Auswählen der Antikörper aus c), die in der Lage sind, die Aktivität des Zielproteins zu modulieren.

16. Verfahren nach Anspruch 15, wobei der Schritt d) das Bestimmen umfasst, ob die angezogenen Antikörper Agonisten, Antagonisten oder allosterische Modulatoren des Zielproteins sind.

17. Verfahren nach einem der Ansprüche 1 bis 16, wobei das Verfahren für ein Kandidatenmolekül durchgeführt wird.

18. Verfahren nach einem der Ansprüche 1 bis 16, wobei das Verfahren für einen Pool von Kandidatenmolekülen durchgeführt wird.

19. Verfahren nach einem der Ansprüche 1 bis 18, wobei das Verfahren mit einem Paar aus einem funktionalen Antikörper/Zielmolekül durchgeführt wird.

20. Verfahren nach einem der Ansprüche 1 bis 18, wobei das Verfahren mit mehreren Paaren aus einem funktionalen Antikörper/Zielmolekül durchgeführt wird.

21. Verfahren nach einem der Ansprüche 1 bis 20, wobei das Verfahren ferner den Schritt des Testens der Fähigkeit des ausgewählten Kandidatenmoleküls umfasst, das Zielmolekül in einer flüssigen Phase zu binden.

22. Verfahren nach einem der Ansprüche 1 bis 21, wobei das Verfahren ferner den Schritt des Testens der Fähigkeit des ausgewählte Kandidatenmoleküls umfasst, mit dem funktionalen Antikörper zu interagieren.

23. Verfahren nach einem der Ansprüche 1 bis 22, wobei die Inhibierung der Bindung des funktionalen Antikörpers an das Zielmolekül durch Inhibitions-ELISA bestimmt wird.

24. Verfahren nach einem der Ansprüche 1 bis 23, wobei das Verfahren ferner den Schritt des Testens der Fähigkeit des ausgewählten Kandidatenmoleküls umfasst, das Zielprotein zu binden.

25. Verfahren nach einem der Ansprüche 1 bis 24, wobei das Verfahren die folgenden Schritte umfasst:
(a) In Kontakt bringen eines Kandidatenmoleküls mit einem funktionalen Antikörper und einem Zielmolekül;
(b) Auswählen des Kandidatenmoleküls, das die Bindung des funktionalen Antikörpers an das Zielmolekül inhibiert;
(c) Testen der Fähigkeit des ausgewählten Kandidatenmoleküls aus Schritt b), das Zielmolekül in einer flüssigen Phase zu binden,
(d) Auswählen des Kandidatenmoleküls, das die Bindung des funktionalen Antikörpers an das Zielmolekül in einer flüssigen Phase inhibiert;
(e) Testen der Fähigkeit des ausgewählten Kandidatenmoleküls aus Schritt d), mit dem funktionalen Antikörper zu interagieren; und
(f) Auswählen des Kandidatenmoleküls/ der Kandidatenmoleküle, das/die den funktionalen Antikörper nicht spezifisch bindet/binden.

26. Verwendung von mindestens einem funktionalen Antikörper und mindestens einem Zielmolekül zum Suchen, Auswählen oder Identifizieren eines Moleküls, das auf das Zielprotein wirkt, wobei der funktionale Antikörper in der Lage ist, die Aktivität des Zielproteins zu modifizieren, und gegen ein Zielprotein gerichtet ist und wobei das Zielmolekül ein Fragment des Zielproteins ist, eine Länge von 5 bis 100 Aminosäureresten aufweist und ein Epitop des Zielproteins umfasst, das durch den funktionalen Antikörper erkannt wird.

## Revendications

1. Méthode pour cribler, sélectionner ou identifier une molécule active sur une protéine cible, dans laquelle la méthode comprend la mise en contact d'une molécule candidate, d'un anticorps fonctionnel ou des anticorps fonctionnels qui est ou sont capable(s) de modifier l'activité de ladite protéine cible, et une molécule cible qui est un fragment de la protéine cible et est longue de 5 à 100 acides aminés, dans laquelle la molécule cible comprend un ou plusieurs épitopes du ou des sites actifs de ladite protéine cible qui est/sont reconnu(s) par ledit ou lesdits anticorps fonctionnel(s), et la détermination de la capacité de ladite molécule candidate à inhiber la liaison dudit ou desdits anticorps fonctionnel(s) sur ladite molécule cible.

2. Méthode selon la revendication 1, dans laquelle ladite molécule cible est longue de 5 à 35 acides aminés.

3. Méthode selon la revendication 1 ou 2, dans laquelle ladite molécule cible comprend ou consiste en une séquence d'acides aminés du fragment peptidique de la protéine cible utilisé comme immungène pour la préparation dudit anticorps fonctionnel.

4. Méthode selon l'une quelconque des revendications précédentes, dans laquelle ledit anticorps fonctionnel ou ladite molécule cible est immobilisé sur un support solide.

5. Méthode selon la revendication 4, dans laquelle ladite molécule cible est immobilisée sur un support solide, soit directement soit en utilisant une molécule espaceur.

6. Méthode selon la revendication 4 ou 5, dans laquelle le support solide est un puit, une bille ou une colonne.

7. Méthode selon la revendication 5 ou 6, dans laquelle la méthode comprend l'étape de:
(a) immobiliser la molécule cible sur un support solide;
(b) mettre en contact la molécule cible immobilisée avec la molécule candidate;
(c) ajouter l'anticorps fonctionnel; et
(d) détecter la liaison dudit anticorps fonctionnel sur ladite molécule cible.

8. Méthode selon l'une quelconque des revendications 1-7, dans laquelle ladite protéine cible est une enzyme.

9. Méthode selon l'une quelconque des revendications 1-7, dans laquelle ladite protéine cible est une protéine membranaire.

10. Méthode selon la revendication 9, dans laquelle ladite protéine membranaire est un récepteur.

11. Méthode selon la revendication 10, dans laquelle ledit récepteur est un récepteur couplé à une protéine G (GPCR).

12. Méthode selon l'une quelconque des revendications 1-11, dans laquelle ledit anticorps est un anticorps monoclonal.

13. Méthode selon l'une quelconque des revendications 1-11, dans laquelle ledit anticorps est un anticorps polyclonal.

14. Méthode selon l'une quelconque des revendications 1-11, dans laquelle ledit anticorps est un fragment d'anticorps tel que Fab, Fab', Fab'2, régions CDR ou un dérivé d'anticorps tel qu'un anticorps simple-chaine, un anticorps humanisé, ou un anticorps poly-fonctionnel.

15. Méthode selon l'une quelconque des revendications précédentes, dans laquelle ledit anticorps fonctionnel est préparé en :
a. sélectionnant au moins un fragment peptidique de la protéine cible ;
b. levant des anticorps à partir dudit peptide sélectionné ;
c. sélectionnant les anticorps levés qui sont capables de se lier au peptide et/ou la protéine cible ; et,
d. sélectionnant les anticorps de c) qui sont capables de moduler l'activité de la protéine cible.

16. Méthode selon la revendication 15, dans laquelle l'étape d) comprend la détermination de la capacité des anticorps levés à être des agonistes, des antagonistes ou des modulateurs allostériques de la protéine cible.

17. Méthode selon l'une quelconque des revendications 1-16, dans laquelle la méthode est effectuée sur une molécule candidate.

18. Méthode selon l'une quelconque des revendications 1-16, dans laquelle la méthode est effectuée sur un ensemble de molécules candidates.

19. Méthode selon l'une quelconque des revendications 1-18, dans laquelle la méthode est effectuée avec un couple d'anticorps fonctionnel/molécule cible.

20. Méthode selon l'une quelconque des revendications 1-18, dans laquelle la méthode est effectuée avec plusieurs couples d'anticorps fonctionnel/molécule cible.

21. Méthode selon l'une quelconque des revendications 1-20, dans laquelle la méthode comprend en outre l'étape de tester la capacité de la molécule candidate sélectionnée à lier la molécule cible en phase liquide.

22. Méthode selon l'une quelconque des revendications 1-21, dans laquelle la méthode comprend en outre l'étape de tester la capacité de la molécule candidate sélectionnée à interagir avec l'anticorps fonctionnel.

23. Méthode selon l'une quelconque des revendications 1-22, dans laquelle l'inhibition de la liaison dudit anticorps fonctionnel à ladite molécule cible est déterminée par un ELISA d'inhibition.

24. Méthode selon l'une quelconque des revendications 1-23, dans laquelle la méthode comprend en outre l'étape de tester la capacité de la molécule candidate sélectionnée à lier la protéine cible.

25. Méthode selon l'une quelconque des revendications 1-24, dans laquelle la méthode comprend l'étape de :
(a) mettre en contact une molécule candidate avec un anticorps fonctionnel et une molécule cible ;
(b) sélectionner la molécule candidate qui inhibe la liaison dudit anticorps fonctionnel à ladite molécule cible ;
(c) tester la capacité de la molécule candidate sélectionnée à l'étape b) à lier la molécule cible en phase liquide ;
(d) sélectionner la molécule candidate qui inhibe la liaison dudit anticorps fonctionnel à ladite molécule cible en phase liquide ;
(e) tester la capacité de la molécule candidate sélectionnée à l'étape d) à interagir avec ledit anticorps fonctionnel ; et
(f) sélectionner la(les) molécule(s) candidate(s) qui ne lie(nt) pas spécifiquement ledit anticorps fonctionnel.

26. Utilisation d'au moins un anticorps fonctionnel qui est capable de modifier l'activité de ladite protéine cible, dirigé contre une protéine cible et au moins une molécule cible qui est un fragment de la protéine cible, longue de 5 à 100 acides aminés et comprenant un epitope de ladite protéine cible qui est reconnu par ledit anticorps fonctionnel pour le criblage, la sélection ou l'identification d'une molécule active sur ladite protéine cible.
